Europäisches Patentamt

European Patent Office

Office européen des brevets

(19)

(11)  EP 0 884 997 B1

(12)  EUROPEAN PATENT SPECIFICATION

(45) Date of publication and mention
of the grant of the patent:
**05.06.2002  Bulletin 2002/23**

(21) Application number: **97903290.1**

(22) Date of filing: **13.02.1997**

(51) Int Cl.7: **A61K 7/06**, A61K 7/48

(86) International application number:
**PCT/EP97/00714**

(87) International publication number:
**WO 97/29733 (21.08.1997 Gazette 1997/36)**

(54) **COMPOSITIONS CONTAINING AN ANTIFUNGAL AND A SULFUR COMPOUND**

FUNGIZIDE UND SCHWEFEL ENTHALTENDE ZUSAMMENSETZUNGEN

COMPOSITIONS CONTENANT UN ANTIFONGIQUE ET UN COMPOSE SOUFRE

(84) Designated Contracting States:
**AT BE CH DE DK ES FI FR GB GR IE IT LI LU NL
PT SE**
Designated Extension States:
**LT LV RO SI**

(30) Priority: **16.02.1996  EP 96200409**

(43) Date of publication of application:
**23.12.1998  Bulletin 1998/52**

(73) Proprietors:
• **JANSSEN PHARMACEUTICA N.V.
2340 Beerse (BE)**
• **NEUTROGENA CORPORATION
Los Angeles California 90045-5544 (US)**
• **Johnson & Johnson Consumer Companies, Inc.
Skillman, New Jersey 08558-9418 (US)**

(72) Inventors:
• **ODDS, Frank, Christopher
B-2340 Beerse (BE)**
• **DE DONCKER, Piet, R., G.
B-2340 Beerse (BE)**
• **HO, Kie
Los Angeles, CA 90045 (US)**
• **FERNANDEZ, Candelario, A.
Los Angeles, CA 90045 (US)**
• **CAUWENBERGH. Gerard Frans Maria Jan
c/o Johnson &
Skillman, NJ 08558-9418 (US)**

(74) Representative: **Quaghebeur, Luc et al
Janssen Pharmaceutica N.V.,
Patent Department,
Turnhoutseweg 30
2340 Beerse (BE)**

(56) References cited:
EP-A- 0 680 745            WO-A-96/29045
WO-A-96/29983            GB-A- 2 058 563
US-A- 4 835 148

• CHEMICAL ABSTRACTS, vol. 117, no. 9, 31
August 1992 Columbus, Ohio, US; abstract no.
90300, J.BARTROLI E.A.: "Process for
preparation of new pyridine oxide-containing
triazole derivatives as fungicides" XP002033182
& ES 2 024 349 A (J.URIACH) 16 February 1992
• CHEMICAL ABSTRACTS, vol. 122, no. 20, 15 May
1995 Columbus, Ohio, US; abstract no. 248020,
C.CASTILLA,A.CARLOS: "Fast-acting
antifungal and antibacterial shampoo "
XP002033183 & ES 2 061 407 A (SPAIN) 1
December 1994

EP 0 884 997 B1

## Description

The invention relates to compositions such as body and hair cleansing products, in particular shampoos, comprising one or more fungal ergosterol biosynthesis inhibiting antifungals as a first active ingredient, a pyrithione salt as a second active ingredient, and art-known body or hair cleansing product ingredients as a carrier.

### Background of the invention

Known medicated shampoos are, for example, the ketoconazole shampoos which are marketed in a 2 % formulation and which show a beneficial effect in seborrheic dermatitis after topical application. Ketoconazole was disclosed by Rosenberg et al. in US-4,569,935 to be useful in the topical treatment of psoriasis and seborrheic dermatitis. Ketoconazole shampoos that exhibit better cosmetic attributes such as lathering and conditioning, and are acceptably stable to degradation so that they can be formulated to contain less than 2 % active ingredient are disclosed in US-5,456,851. Elubiol shampoos having a skin grease regulating action besides an antifungal action are known from WO-93/18743. Some anti-dandruff formulations contain coal tar, selenium sulfide or a pyrithione salt, e.g. zinc or sodium pyrithione as an active agent. WO-96/29045 generically discloses combinations of such a cytotoxic agent and an antifungal agent for the treament of seborrheic dermatitis of the scalp ; specifically disclosed is the combined use of an unidentified composition comprising 1.8 % coal tar and an unidentified solution comprising 2 % ketoconazole. WO-96/29983 discloses mild aqueous detergent compositions comprising from about 4 to about 12 % by weight of an anionic surfactant, an amphoteric surfactant at a level of at least about 0.75 parts by weight per part by weight of said" anionic surfactant, and one or more of 11 listed therapeutic agents. Still other anti-dandruff shampoos contain selenium sulfide which is toxic when taken by mouth or applied to extensive areas of the skin. ES-2,061,407 (Chem. Abstr., vol 122, no. 20 15.05.1995, abstract no 248020) discloses a shampoo composition comprising shampoo base, zinc pyrithione, econazole nitrate, salicylic acid, neomycin undecenylate, chlorhexidine, vitamin A and malachite green dye.

Prior art shampoos comprising anti-dandruff agents are designed in such a way that an optimum balance is achieved between efficacy and tolerability; the concentration of the active ingredient in the medicated shampoos is such that as many users as possible are effectively treated and as few as possible suffer adverse effects. Nonetheless, there remain substantial numbers of patients who do not benefit from using prior art shampoos, either because they do not respond to the treatment, or worse, because they do not tolerate the treatment with a particular medicated shampoo.

The number of patients not responding to particular medicated shampoo can be quite high (ketoconazole up to 30 % ; selenium sulfide up to 40 %). Consequently, there is a hard felt need for new shampoos which provide effective anti-dandruff treatment for a larger proportion of number of patients using such a new shampoo ; i.e. a new shampoo for which there are fewer non-respondents than with prior art shampoos.

On the other hand, patients suffering from dandruff or seborrheic dermatitis, as well as the authorities approving medicated shampoos, apply increasingly stricter criteria which such shampoos should meet. Amongst these criteria the most important are : absence of further aggravation of the condition due to the treatment, lowest possible incidence of side effects, further increase in the absence of symptoms such as irritation, pruritus and scaling (both adherent and loose scaling) ; improved cosmetic acceptability, in particular, good cleansing properties, absence of odour or stench, absence of staining or soiling of the clothes, and overall conditioning (wet and dry combing properties). Dandruff or seborrheic dermatitis are often accompanied by high or excessive oil or sebum production, and compositions having a beneficial effect thereon would clearly constitute a further advance in the treatment of dandruff.

Thus far, in order to achieve the above desiderata, most efforts have involved reformulating the shampoo base. There is, however, still a need for increasing the tolerability / acceptability of medicated shampoos, i.e. new shampoos are desired that are tolerated better by larger proportions of patients using such new shampoos.

### Description of the invention

The present invention relates to compositions such as body and hair cleansing products, in particular shampoos, comprising, consisting essentially of or consisting of one or more fungal ergosterol biosynthesis inhibiting antifungals as a first active ingredient, a pyrithione salt as a second active ingredient, and art-known body and hair cleansing product ingredients as a carrier. In the following description, the invention is illustrated using shampoos as examples, but it will be evident to a person skilled in the art that the combinations according to the present invention can be utilized just as well in other body and hair cleansing products.

The combination of two differently acting anti-dandruff agents has two distinct advantages over the prior art shampoos which contain either of the active ingredients alone. First, an increased proportion of patients suffering from dandruff or seborrheic dermatitis respond to the shampoos according to the present invention. Secondly, some combinations act synergistically and as a consequence thereof, the concentration of one or both of the different types of agent can be lowered, thus increasing the tolerability. Each class of ingredients will now be discussed in turn.

The fungal ergosterol biosynthesis inhibiting antifungal is ketoconazole or elubiol. The azole compounds ketoconazole and elubiol harm the normal flora of the skin, in particular of the scalp, the least. Ketoconazole and elubiol produce a mutual synergistic effect on dermatophyte fungi when in used in combination with a pyrithione salt (vide infra). Effective amounts of the antifungals in compositions according to the present invention are in the range of from about 0.1 % to about 2 % (w/w), and preferably from about 0.5 % to about 1 % (w/w). As will be explained further, at the lower end of this range, special precautions may have to be taken in order to ensure that the shampoo does not lose its efficacy due to degradation of the antifungal compound upon storage. Concentrations higher than those indicated do not improve the treatment of the conditions any further, and are on the whole more detrimental than beneficial.

The second active ingredient, pyrithione, may be present in an amount ranging from about 0.05 % to about 2 % (w/w), and preferably from about 0.3 % to about 1% (w/w). One skilled in the art will readily recognize that the nature of the particular pyrithione salt form has an effect on the amount when expressed as % (w/w). Therefore, the above-mentioned amounts, and all following references to amounts of pyrithione, are expressed for zinc pyrithione [MW = 317.7]. Zinc pyrithione and sodium pyrithione are preferred pyrithione salts.

Preferably, the first and the second active ingredients are present in quantities producing a mutual synergistic effect on the inhibition of the growth of dermatophyte fungi, in particular the species associated with dandruff and seborrheic dermatitis, i.e. *Malessezia* (*Pityrosporum*) *ovale, M. furfur*, but also other species such as *Epidermophyton, Microsporum, Trichophyton* associated with, for example, microsporosis, tinea capitis, pityriasis versicolor and the like. The ratio of the quantities of the first and the second active ingredient will depend on the nature of said active ingredients and on the target species. Particularly, it is contemplated that the weight: weight ratio between the first and the second active ingredient (antifungal : pyrithione) may range from about 5 : 1 to about 1 : 5, in particular form about 2 : 1 to about 1 : 2. For example, and as already mentioned, ketoconazole and elubiol when in used in combination with a pyrithione salt, in particular when used in similar quantities such as in a weight ratio ranging from about 2 : 1 to about 1 : 2, in particular in a weight range of about 1 : 1, produce a mutual synergistic effect on dermatophyte fungi, in particular on *Malessezia* (*Pityrosporum*) *ovale.* Unexpectedly, these combinations also produce a mutual synergistic effect on the pathogenic yeast *Candida albicans.*

The shampoos according to the present invention may comprise further anti-dandruff agents such as, for example, selenium sulfide, pioctone or ciclopirox olamine.

The shampoos according to the present invention can conveniently be formulated using art-known shampoo bases ; the art-known shampoo ingredients comprise one or more of a surfactant, a foaming agent, a thickener sufficient to give the final formulation a viscosity in the range of 4,000 to 9,000 mPa.s at room temperature, a preservative, an anti-oxidant, and acid or base or buffer sufficient to give the shampoo a pH in the range of from about 4 to about 10. A single ingredient can have two or more functions, e.g. surfactant and foaming agent, or anti-oxidant and buffer.

Suitable surfactants for use in the shampoos according to the present invention may be selected from the group comprising sodium $C_{14-16}$ olefin sulfonates, sodium lauryl sulfate, TEA lauryl sulfate, sodium laureth sulfate, cocamidopropylamine oxide, lauryl amine oxide, lauramido DEA, cocamidopropyl betaine, lauryl dimethyl betaine, cocodimethyl sulfo-propyl betaine, sodium cocoyl sarcosinate, disodium oleamido MIPA sulfosuccinate, disodium cocamido MIPA sulfosuccinate, disodium laureth sulfosuccinate, cocoamphocarboxy-glycinate, disodium oleamido MEA sulfosuccinate, amine glycinates, amine propionates and amine sultaines, and mixtures thereof. Preferably, a mixture of two or more different surfactants , in particular sodium laureth sulfate and sodium cocoyl sarcosinate, or sodium lauryl sulfate, sodium laureth sulfate, TEA lauryl sulfate and cocamidopropyl betaine, may be used in the present shampoos. In the shampoos according to the present invention, the total amount of surfactants may range from about 36% to about 45% (w/w). Preferably, the weight of amphoteric surfactants is less than 15 % by weight of the total amount of surfactants.

In the above definitions, and hereinafter, the term 'MEA' signifies a monoethanolamide of formula $RCO-NH-CH_2CH_2-OH$, the term 'DEA' signifies di-ethanol amide of formula $RCO-N(CH_2CH_2-OH)_2$, 'TEA' signifies triethanolammonium ; the term 'MIPA' signifies a mono-isopropanol amide of formula $RCO-NH-CH_2-CHOH-CH_3$ ; wherein each RCO-group is a fatty acid residue, such as a $C_{13-19}$alkylcarbonyl or $C_{13-19}$alkenylcarbonyl group.

Suitable foaming agents (foam boosters and stabilizers) for use in the shampoos according to the present invention may be selected from the group of fatty acid mono-and dialkanol-amides comprising cocamide MEA, cocamide DEA, oleamide MEA, oleamide DEA and mixtures thereof. The foaming agent may be present in a range from about 1 to about 10 % (w/w), preferably from about 2 to about 6 % (w/w), in particular about 4 to about 5 % (w/w). These ingredients typically also have a thickening effect on the formulation.

Suitable preservatives for use in the present shampoos are dermatologically acceptable preservatives, e.g. tetrasodium EDTA, methylparaben, propylparaben, butylparaben, ethylparaben, imidazolidinyl urea, phenoxyethanol, quaternium 15, citric acid, preferably in combinations with one another. Tetrasodium EDTA and citric acid also function as chelating agents.

As disclosed in US-5,456,851, when the concentration of ketoconazole, or for that matter that of any other antifungal, is at the lower end of the ranges mentioned hereinabove, the addition of a carefully controlled amount of an antioxidant selected from the group consisting of butylated hydroxytoluene ("BHT"), butylated hydroxyanisole ("BHA"), ascorbic acid and N-acetyl-cysteine effectively stabilizes the ketoconazole or other azole present in the shampoo against degradation during accelerated aging for 13 weeks at 50°C, which is considered to be predictive of performance during storage at ambient temperatures for two years. Effective stability is considered to be a loss of active ingredient during storage of not more than about 10 percent. The proportion of BHT or BHA that has been found to be most effective is within the range of from about 0.01 % to about 1 % (w/w). Proportions greater than this amount do not stabilize ketoconazole as effectively for the 13-week accelerated aging period, although if one extends the accelerated aging period longer than 13 weeks, greater proportions of BHT or BHA tend to be more effective, since the BHT or BHA itself is also subject to degradation. However, it is well recognized by government regulatory agencies and in the pharmaceutical and cosmetic industries that stability testing for 13 weeks at 50°C is quite sufficient to predict product stability during normal shelf life storage for two (2) years at room temperature. It is also equally important that, for safety reasons (that is, to minimize the potential for skin sensitization), it is desired to use as small an amount as possible of BHT or BHA.

Since shampoo users expect a shampoo to be slightly viscous, one or more thickeners are often included in the formulation which give it a viscosity in the range of 4,000 to 9,000 mPa.s at room temperature. A suitable thickener is a carbomer or polycarboxylic acid, such as Carbopol ™ 1342, which is thickened by the addition of sodium hydroxide or sodium chloride at the end of the preparation. Other suitable thickeners are the foaming agents mentioned hereinabove, preferably cocamide MEA.

The shampoo may further comprise a conditioner such as polyquaternium-7 or a similar cationic quaternary polymer, e.g. a quaternary silicone polymer ; one or more pearlizing agents selected from the group consisting of ethylene glycol distearate, ethylene glycol monostearate and mixtures thereof; and/or one or more fragrances and one or more colorants.

The pH of the shampoos according to the present invention are conveniently established using dermatologically acceptable acids, bases and buffers. The pH can range from about 4 to about 10, but preferably is in the range of about 6.5 to about 8, in particular from about 6.9 to about 7.4.

Some pyrithione salts, as well as the some of the first active ingredients when at approximately neutral pH (pH 6 to 8), have limited solubility. In order to keep these agents homogeneously distributed throughout the shampoo, a suspending agent such as, for example, Avicel RC-591™ (a mixture of sodium CMC and microcrystalline cellulose) may be added. Several of the shampoo base ingredients, however, have considerable suspending properties of their own, and therefore the inclusion of particular suspending agents in the present shampoos is entirely optional.

The components of the shampoo are employed in conventional amounts, for example:

(a) 36% to 45% surfactants,
(b) 2% to 6% foaming agent.
(c) 0.1% to 2% antifungal,
(d) 0.05 to 2 % pyrithione salt
(e) 0.2% to 1.3 % thickener,
(f) 0.01% to 1% BHT or BHA;
(g) preservatives sufficient to retard degradation of the final composition in order to give adequate shelf life,
(h) acid, base or buffer to yield a pH in the desired range, and
(i) water qs ad 100% (that is, sufficient water to make 100%).

Examples

In the following, a general process for preparing shampoos according to the present invention is presented. Suitable amounts for each of the ingredients can be derived from the preceding description and from the exemplary formulations shown in the tables hereinafter.

A vessel was charged with a 1.64% stock solution of Carbopol 1342 (prepared using a Quadro disperser which functions by keeping the powdered polymer evenly divided and pulling the powder by a vacuum into a stream of water) and deionized water, and heated to about 70°C. Both surfactants, i.e. sodium laureth sulfate and sodium cocoyl sarcosinate, were added, followed by the foaming agent, cocamide MEA, and a pearlizing agent (ethylene glycol distearate) and mixed until complete dissolution. Then the BHT was added and the mixture was stirred until complete dissolution thereof. The solution was allowed to cool slightly, whereupon the antifungal ingredient was added while stirring well. (The antifungal is added while the pH is slightly acidic, to facilitate dissolution.) Next, a pyrithione salt was dispersed into the mixture and stirred until homogenously dispersed. The mixture was allowed to cool to about 40°C, at which temperature there were added the conditioner (polyquaternium-7), the preservatives quaternium-15 and tetrasodium EDTA, the colorants and fragrances, and the NaCl for thickening the solution. The pH of the solution was adjusted to 6.9-7.4 with a 25% aq. solution of NaOH and deionized water was added to the final volume. Similar shampoo formulations can prepared using analogous processes which will be apparent to the person skilled in the art.

While the above process in general is satisfactory, it has the disadvantage of forming a pinkish color during the process at the stage where the ketoconazole is dissolved in the shampoo base ; in the finished product the color may disappear or fade when the pH is adjusted to 6.9-7.4, but when the finished product is subjected to stability tests under stress (at an elevated temperature) the pink color reappears. Investigation of this problem has unexpectedly shown that not the temperature, but the pH range at which the active ingredient is dissolved is critical ; even slightly acidic conditions appear to be causing some color formation. While the rate of dissolution of ketoconazole in basic conditions is slow, a satisfactory compromise between preventing color formation and a sufficiently rapid rate of dissolution can be attained by dissolving ketoconazole in the pH range of 6.9 to 7.3. Shampoos obtainable by the improved process have several advantages : they do not any longer require the addition of artificial colorants ; they have improved long-term stability since they do not change color with time ; and for the first time they enable one to make clear, white formulations.

In the following, two improved processes for preparing shampoos without colorants are presented.

Example A : White-Pearlescent Ketoconazole-Containing Shampoo (Ref. #780-135)

| Ingredient | Percent |
|---|---|
| purified water | 50.72 |
| carbopol 1342 NF | 0.60 |
| sodium laureth sulfate | 30.00 |
| sodium cocoyl sarcosinate | 10.00 |
| cocamide MEA | 4.00 |
| ethylene glycol distearate | 1.25 |
| sodium hydroxide | 0.23 |
| BHT | 0.10 |
| ketoconazole | 1.00 |
| tetrasodium EDTA | 0.50 |
| perfume oil | 0.30 |
| quaternium-15 | 0.05 |
| polyquaternium-7 | 1.00 |
| sodium chloride | 0.25 |
| | 100.00 |

Process description for white-pearlescent ketoconazole-containing shampoo

1. Load purified water in the vessel. Start the homogenizer. Set the propeller speed at 10 rpm. Start mixing at an internal pressure of 0.6 bar.
2. Charge Carbopol 1342 from the bottom of the vessel. Regulate internal pressure at 0.3 bar. Continue mixing at 10 rpm for 20 minutes.
3. Charge sodium laureth lulfate and lodium cocoyl sarcosinate from the bottom of the vessel. Start heating to 63°C to 67°C. Maintain internal pressure at 0.6 bar and propeller speed at 10 rpm.
4. Stop the homogenizer. Bring vessel back to the atmospheric pressure and continue mixing at 10 rpm until temperature reaches 63°C to 67°C.
5. At 63°C to 67°C, regulate the pressure of the vessel to 0.6 bar. Start the homogenizer and continue mixing with a propeller speed at 10 rpm. Add cocamide MEA.
6. Maintain temperature at 63°C to 67°C. Stop the homogenizer and continue mixing at 10 rpm until there are no more undissolved particles.
7. Bring internal pressure to 0.5 bar. Add ethylene glycol distearate from the bottom of the vessel. Maintain temperature at 63°C to 67°C. Continue mixing with the propeller speed at 10 rpm for 5 minutes.
8. Continue mixing with the propeller speed at 10 rpm and an internal pressure of 0.6 bar. Adjust pH to 7.0 to 7.3 by adding sodium hydroxide. Continue mixing for 5 minutes.

9. Start the homogenizer. Continue mixing with the propeller speed at 10 rpm and an internal pressure of 0.6 bar. Add BHT and ketoconazole by suction.

10. Maintain temperature at 63°C to 67°C. Continue mixing with the propeller speed at 10 rpm and homogenizer at 1000 rpm for 40 to 60 minutes. Take samples at 15 minute intervals and check through sieve and microscope.

11. Stop the homogenizer. Continue mixing with the propeller speed at 10 rpm. Start cooling to 40°C to 45°C.

12. At 40°C to 45°C, add tetrasodium EDTA, quaternium-15 and perfume oil through the manhole. Continue mixing with only the propeller at 10 rpm. Continue cooling.

13. At 25°C to 30°C, add polyquaternium-7 through the manhole. Continue mixing with the propeller at 10 rpm.

14. Bring internal pressure to 0.8 bar. Adjust to the desired pH (if necessary) by adding hydrochloric acid by suction.

15. Bring the vessel to atmospheric pressure. Stop the homogenizer and mix with the propeller at 10 rpm for 10 minutes.

Stability test results for white-pearlescent ketoconazole-containing shampoo

Three Batches were made with a slight processing change. Samples were placed on stability testing.

| Reference | Process Change | Color |
|---|---|---|
| #780-134 | Ketoconazole added at 66°C and pH 5.6 and mix for 80 min. | Pink |
| #780-135 | Ketoconazole added at 66°C and pH 7.0 and mix for 80 min. | White |
| #780-137 | Ketoconazole added at 40°C and pH 7.0 and mix for 40 min. | White |

| Reference | Process Change | Keto Assay | Color |
|---|---|---|---|
| #9601001 | Ketoconazole added at 67°C and pH 5.0 and mix for 80 min. | 1.03% | Light Pink |
| #9601002 | Ketoconazole added at 67°C and pH 7.2 and mix for 80 min. | 1.07% | White |
| #9601003 | Ketoconazole was dissolved in the surfactant system for 80 min. at 67°C. NaOH was added and mix for 15 minutes (pH=12.5) and then Carbopol Phase was added. | 1.07% | White |
| #9601004 | Ketoconazole was dissolved in the surfactant system for 80 min. at 67°C. Carbopol Phase was then added immediately followed by NaOH and mixed for 40 min. | 1.05% | White |

Example B : Clear ketoconazole-containing shampoo (Ref. #780-115)

| Ingredient | Percent |
|---|---|
| purified water | 42.22 |
| sodium laureth sulfate | 45.00 |
| sodium cocoyl sarcosinate | 5.00 |
| cocamide MEA | 3.50 |
| BHT | 0.10 |
| ketoconazole | 1.00 |
| tetrasodium EDTA | 0.50 |
| perfume oil | 0.30 |
| quaternium-15 | 0.05 |
| polyquaternium-7 | 1.00 |
| hydrochloric acid (6N) | 0.35 |
| sodium chloride | 1.00 |
| | 100.00 |

Process description for clear ketoconazole-containing shampoo.

1. Load purified water in the vessel. Set the propeller speed at 10 rpm. Set the propeller speed at 10 rpm. Start mixing at an internal pressure of 0.6 bar.

2. Charge sodium laureth sulfate and sodium cocoyl sarcosinate from the bottom of the vessel. Start heating to 63°C to 67°C. Maintain internal pressure at 0.6 bar and propeller speed at 10 rpm.

3. Stop the homogenizer. Bring vessel back to the atmospheric pressure and continue mixing at 10 rpm until temperature reaches 63°C to 67°C.

4. At 63°C to 67°C, regulate the pressure of the vessel to 0.6 bar. Start the homogenizer and continue mixing with a propeller speed at 10 rpm. Add cocamide MEA.

6. Maintain temperature at 63°C to 67°C. Stop the homogenizer and continue mixing at 10 rpm until there are no more undissolved particles.

7. Start the homogenizer. Continue mixing with the propeller speed at 10 rpm and an internal pressure of 0.6 bar. Add BHT and ketoconazole by suction.

8. Maintain temperature at 63°C to 67°C. Continue mixing with the propeller speed at 10 rpm and homogenizer at 1000 rpm for 40 to 60 minutes. Take samples at 15 minute intervals and check through sieve and microscope.

9. Stop the homogenizer. Continue mixing with the propeller speed at 10 rpm. Start cooling to 40°C to 45°C.

10. At 40°C to 45°C, add tetrasodium EDTA, quaternium-15 and perfume oil through the manhole. Continue mixing with only the propeller at 10 rpm. Continue cooling.

11. At 25°C to 30°C, add polyquaternium-7 through the manhole. Continue mixing with the propeller at 10 rpm.

12. Bring internal pressure to 0.8 bar. Adjust to the desired pH (if necessary) by adding hydrochloric acid by suction.

13. Bring the vessel to atmospheric pressure. Stop the homogenizer and mix with the propeller at 10 rpm for 10 minutes.

Stability test results for clear light yellow ketoconazole-containing shampoo.

Two Batches were made with a slight processing change. Samples were placed on stability testing.

| Reference | Process Change | Color |
|---|---|---|
| #780-115 | Ketoconazole added at 66°C and pH 8.2 and mix for 40 min. | Clear Light Yellow |
| #780-138 | Ketoconazoie added at 40°C and pH 8.2 and mix for 40 min. | Clear Light Yellow |

Using the general and the improved procedures described above, the following shampoo formulations according to the present invention can be made ; all quantities hereinafter are parts by weight.

The formulations according to the present invention are useful in the treatment of disorders such as dandruff, seborrheic dermatitis, the control of psoriasis, the reduction of oil or sebum production of the scalp, and the like disorders and discomforts. The formulations are to be applied topically to the affected body parts at regular intervals, in particular from at least once weekly to about once daily. Preferably they are employed more often in the beginning of the treatment, e.g. from 4 to 7 times a week, and less frequently in a later stage when the desired effect has been obtained and relapse is to be prevented (e.g. once or twice a week).

Example 1: Shampoo formulations for normal hair

| Ingredients | (a) | (b) |
|---|---|---|
| sodium laureth sulfate | 30 | 30 |
| sodium cocoyl sarcosinate | 10 | 10 |
| cocamide MEA | 4 | 4 |
| ketoconazole USP | 0.5 | 1 |
| pyrithione salt | 0.5 | 1 |
| glycol distearate | 1.25 | 1.25 |
| polyquaternium-7 | 1 | 1 |
| Carbopol™1342 | 0.6 | 0.6 |
| tetrasodium EDTA | 0.5 | 0.5 |
| perfume oil | 0.5 | 0.5 |

(continued)

| Ingredients | (a) | (b) |
|---|---|---|
| sodium chloride | 0.3 | 0.3 |
| sodium hydroxide 25% | 0.92 | 0.9 |
| butylated hydroxytoluene | 0.1 | 0.1 |
| quaternium-15 | 0.05 | 0.05 |
| colorants | 0.001 | 0.001 |
| deionized water qs ad | 100 | 100 - |

Example 2: Shampoo formulations for oily hair

| Ingredients | (a) | (b) | (c) |
|---|---|---|---|
| sodium laureth sulfate | 33.33 | 33.33 | 33.33 |
| sodium cocoyl sarcosinate | 11 | 11 | 11 |
| cocamide MEA | 4 | 4 | 4 |
| ketoconazole USP | 0.5 | 0.75 | 1.2 |
| pyrithione salt | 0.5 | 0.25 | 0.8 |
| glycol distearate | 1.25 | 1.25 | 1.25 |
| polyquaternium-7 | 0.6 | 0.6 | 0.6 |
| Carbopol™ 1342 | 0.75 | 0.75 | 0.75 |
| tetrasodium EDTA | 0.5 | 0.5 | 0.5 |
| perfume oil | 0.5 | 0.5 | 0.5 |
| sodium chloride | 0.3 | 0.3 | 0.3 |
| sodium hydroxide 25% | 1.18 | 1.243 | 1.18 |
| butylated hydroxytoluene | 0.1 | 0.1 | 0.1 |
| quaternium-15 | 0.05 | 0.05 | 0.05 |
| colorants | 0.0053 | 0.0053 | 0.0053 |
| deionized water qs ad | 100 | 100 | 100 |

Example 3: Shampoo formulations for dry or damaged hair

| Ingredients | (a) | (b) | (c) |
|---|---|---|---|
| sodium laureth sulfate | 30 | 30 | 30 |
| sodium cocoyl sarcosinate | 10 | 10 | 10 |
| cocamide MEA | 4 | 4 | 4 |
| ketoconazole USP | 0.75 | 0.33 | 1 |
| pyrithione salt | 0.25 | 0.67 | 1 |
| glycol distearate | 1.25 | 1.25 | 1.25 |
| polyquaternium-7 | 5 | 5 | 5 |
| Carbopol™ 1342 | 0.5 | 0.5 | 0.5 |
| tetrasodium EDTA | 0.5 | 0.5 | 0.5 |
| perfume oil | 0.5 | 0.5 | 0.5 |
| sodium chloride | 0.4 | 0.4 | 0.3 |
| sodium hydroxide 25% | 0.7333 | 0.733 | 1.19 |
| butylated hydroxytoluene | 0.1 | 0.1 | 0.1 |
| quaternium-15 | 0.05 | 0.05 | 0.05 |
| colorants | 0.0018 | 0.0018 | 0.0018 |

(continued)

| Ingredients | (a) | (b) | (c) |
|---|---|---|---|
| deionized water qs ad | 100 | 100 | 100 |

In all the formulations given above in Examples 1-3, the proportion of sodium hydroxide may vary slightly, to arrive at the preferred pH level of 6.9 to 7.4, and the proportion of salt (NaCl) may vary, to arrive at the desired viscosity. Formulations prepared according to the improved process and wherein the colorants have been omitted, have an off-white pearlescent look.

Example 4 : Combination of Zinc Pvrithione and Ketoconazole (Ref #822-076)

| Ingredients | Percent |
|---|---|
| purified water | 44.30 |
| sodium laureth sulfate | 15.00 |
| sodium lauryl sulfate | 10.00 |
| TEA lauryl sulfate | 12.00 |
| Zinc Omadine (48%) | 2.10 |
| ketoconazole | 1.00 |
| methylparaben | 0.20 |
| propylparaben | 0.05 |
| cocamide MEA | 5.00 |
| ethylene glycol distearate | 1.25 |
| polyquaternium-7 | 3.00 |
| imidazolidinyl urea | 0.50 |
| cocamidopropyl betaine | 5.00 |
| citric acid | 0.35 |
| fragrance | 0.25 |
| | 100.00 |

Example 5 : Combination of Zinc Pyrithione and Elubiol (Ref #822-075)

| Ingredients | Percent |
|---|---|
| purified water | 44.30 |
| sodium laureth sulfate | 15.00 |
| sodium lauryl sulfate | 10.00 |
| TEA lauryl sulfate | 12.00 |
| Zinc Omadine (48%) | 2.10 |
| elubiol | 1.00 |
| methylparaben | 0.20 |
| propylparaben | 0.05 |
| cocamide MEA | 5.00 |
| ethylene glycol distearate | 1.25 |
| polyquaternium-7 | 3.00 |
| imidazolidinyl urea | 0.50 |
| cocamidopropyl betaine | 5.00 |
| citric acid | 0.35 |
| fragrance | 0.25 |
| | 100.00 |

In the formulations given above in Examples 4 and 5, the proportion of citric acid may vary slightly, to arrive at the preferred pH level of 6.9 to 7.4. The formulations were prepared according to the improved process and have an white-pearlescent look.

Example 6 : MIC of Shampoos against *Pityrosporum ovale* and *Candida albicans*

The shampoos of examples 4 and 5, a shampoo base lacking the active ingredients and two shampoo bases each containing just one of the two active ingredients, were investigated for their in vitro activity against five strains of *Pityrosporum ovale* ATCC numbers 42132, 44337, 44340, 44342 and 44343 and one strain of *Candida albicans* H 29. The shampoos were diluted directly into the culture medium, Diagnostic Sensitivy Test agar (DST ; Oxoid, UK) with the addition of Tween 80 (2 ml/l) and glyceryl monostearate (2.5 g/l) to give concentrations of 0.1 to 40 % of the shampoos in the culture medium. Consequently, the minimum inhibitory concentration (MIC) values are for the whole shampoo, and not only the active ingredient(s). The MIC is the lowest concentration of the shampoo at which a total inhibition of growth was observed. The microorganisms were added on the agar medium in concentrations of $10^7$ cell/ml (*P. ovale*) and $10^6$ cells/ml (*C. albicans*). Plates were incubated at 37°C and read after 1, 2 and 3 days. All shampoos were tested in duplicate.

| Shampoo of Example 4 containing 1% Ketoconazole and 1% Zinc Pyrithione (ZPT) | | | | | | |
|---|---|---|---|---|---|---|
| | *P. ovale* ATCC 42132 | *P. ovale* ATCC 44337 | *P. ovale* ATCC 44340 | *P. ovale* ATCC 44342 | *P.o vale* ATCC 44343 | *C. albicans* H. 29 |
| Shampoo. Base | 5.0% | 5.0% | 5.0% | 5.0% | 5.0% | 5.0% |
| Base + 1% Ketoconazole | 0.5% | 0.5% | 0.5% | 0.5% | 0.5% | 0.5% |
| Base + 1% ZPT | 2.5% | 2.5% | 2.5% | 2.5% | 2.5% | 5.0% |
| Base + 1% Ketoconazole + 1% ZPT | 0.1% | 0.1% | 0.1% | 0.1% | 0.1% | 0.1% |

The base containing 1% Ketoconazole + 1% ZPT is 5 times more effective than the base with 1% Ketoconazole or the base with 1% ZPT, indicating synergistic effect.

| Shampoo of Example 5 containing 1% Elubiol and 1% Zinc Pyrithione (ZPT) | | | | | | |
|---|---|---|---|---|---|---|
| | *P. ovale* ATCC 42132 | *P. ovale* ATCC 44337 | *P. ovale* ATCC 44340 | *P. ovale* ATCC 44342 | *P. ovale* ATCC 44343 | *C. albicans* H. 29 |
| Shampoo. Base | 5.0% | 5.0% | 5.0% | 5.0% | 10.0% | 20.0% |
| Base + 1% Elubiol | 1.0% | 1.0% | 1.0% | 1.0% | 1.0% | 5.0% |
| Base + 1% ZPT | 2.5% | 2.5% | 2.5% | 2.5% | 2.5% | 5.0% |
| Base + 1% Elubiol + 1% ZPT | 0.1% | 0.1% | 0.1% | 0.1% | 0.1% | 0.1% |

The base containing 1% Elubiol + 1% ZPT is 10 times more effective than the base with 1% Elubiol or the base with 1% ZPT, indicating synergistic effect.

**Claims**

1.　A body or hair cleansing composition comprising

(a-1) ketoconazole or elubiol as first active ingredient,
(a-2) a pyrithione salt as a second active ingredient, and
(b) art-known body or hair cleaning product ingredients as a carrier, wherein the first and the second active ingredients are present in quantities producing a mutual synergistic effect on the inhibition of the growth of dermatophyte fungi.

2. A composition according to claim 1 wherein pyrithione salt is zinc pyrithione or sodium pyrithione.

3. A composition according to claim 1 or 2 wherein the first active ingredient is present in an amount ranging from about 0.1% to about 2% (w/w) and the second active ingredient is present in an amount ranging from about 0.05% to about 2% (w/w), the amount of the latter being expressed as weight of zinc pyrithione.

4. A composition according to any one of the preceding claims formulated as a shampoo.

5. A shampoo according to claim 4 wherein the art-known shampoo ingredients comprise one or more of a surfactant, a foaming agent, a thickener sufficient to give the final formulation a viscosity in the range of 4,000 to 9000 mPa. s at room temperature, a preservative, an anti-oxidant, and acid or base or buffer sufficient to give the shampoo a pH in the range of from about 4 to 10.

6. A shampoo according to claim 5 comprising one or more surfactants selected from the group comprising sodium C14-16 olefin sulfonates, sodium lauryl sulfate, sodium laureth sulfate, cocamidopropylamine oxide, lauryl amine oxide, lauramido DEA, cocamidopropyl betaine, lauryl dimethyl betaine, cocodimethyl sulphopropyl betaine, sodium cocoyl sarcosinate, disodium oleamido MIPA sulfosuccinate, disodium cocamido MIPA sulfosuccinate, disodium laureth sulfosuccinate, cocoamphocarboxy-glycinate, disodium oleamido MEA sulfosuccinate, amine glycinates, amine propionates and amine sultaines, and mixtures thereof.

7. A shampoo according to claim 5 wherein the foaming agent is selected from the group of fatty acid mono-and di-alkanolamides consisting of cocamide MEA, cocamide DEA, oleamide MEA, oleamide DEA and mixtures thereof.

8. A shampoo according to claim 5 wherein the antioxidant is butylated hydroxytoluene or butylated hydroxyanisole employed in an amount of about 0.01 to about 1% (w/w).

9. A shampoo according to claim 5 further comprising a conditioner.

10. A shampoo according to claim 5 further comprising one or more pearlizing agents selected from the group consisting of ethylene glycol distearate, ethylene glycol monostearate and mixtures thereof.

11. A shampoo according to claim 5 further comprising one or more fragrances and one or more colorants.

12. A process for preparing a shampoo formulation as defined in any one of the preceding claims comprising the steps of:

(a) heating a solution of thickener and deionized water,
(b) mixing the surfactants, the foaming agent and optionally the pearlizing agent with solution of (a),
(c) mixing the BHT with the solution of (b),
(d) mixing the antifungal with the solution of (c),
(e) dispersing the pyrithione salt in the mixture of(d),
(f) allowing the suspension of (e) to cool somewhat and mixing therewith the preservative(s), the sodium chloride for thickening to the required viscosity, and optionally the conditioner, the fragrance(s) and colorant(s),
(g) adding acid, base or buffer to the solution of (f) to yield a pH in the range of 4 to 10, and
(h) adding deionized water to the solution of (g) to 100%.

**Patentansprüche**

1. Körper- oder Haarreinigungszusammensetzung mit
(a-1) Ketoconazol oder Elubiol als erstem Wirkstoff,
(a-2) einem Pyrithionsalz als zweitem Wirkstoff, sowie

(b) fachbekannten Körper- oder Haarreinigungsmittelbestandteilen als Träger, wobei der erste und zweite Wirkstoff in solchen Mengen vorliegt, daß eine gegenseitige synergistische Wirkung auf die Wachstumshemmung von pilzlichen Dermatophyten ausgeübt wird.

2. Zusammensetzung nach Anspruch 1, wobei es sich bei dem Pyrithionsalz um Zink-Pyrithion oder Natrium-Pyrithion handelt.

3. Zusammensetzung nach Anspruch 1 oder 2, wobei der erste Wirkstoff in einer Menge von ungefähr 0,1% bis ungefähr 2% (w/w) und der zweite Wirkstoff in einer Menge von ungefähr 0,05% bis ungefähr 2% (w/w), wobei letztere Menge als Gewicht an Zink-Pyrithion ausgedrückt wird, vorliegt.

4. Zusammensetzung nach einem der vorhergehenden Ansprüche als Shampooformulierung.

5. Shampoo nach Anspruch 4, bei dem die fachbekannten Shampoobestandteile einen oder mehrere der folgenden Bestandteile umfassen: ein Tensid, einen Schaumbildner, ein Verdickungsmittel in solch einer Menge, daß er der Endformulierung eine Viskosität im Bereich von 4 000 bis 9 000 mPa.s bei Raumtemperatur verleiht, ein Konservierungsmittel, ein Antioxidans, sowie eine Säure oder Base oder ein Puffer in solch einer Menge, daß sie bzw. er dem Shampoo einen pH im Bereich von ungefähr 4 bis 10 verleiht.

6. Shampoo nach Anspruch 5 mit einem Tensid bzw. mehreren Tensiden aus der Gruppe Natrium-C14-16-olefinsulfonate, Natriumlaurylsulfat, Natriumlaurethsulfat, Cocamidopropylaminoxid, Laurylaminoxid, Lauramido-DEA, Cocamidopropylbetain, Lauryldimethylbetain, Cocodimethylsulfopropylbetain, Natriumcocoylsarcosinat, Dinatriumoleamido-MIPAsulfosuccinat, Dinatriumcocamido-MIPA-sulfosuccinat, Dinatriumlaurethsulfosuccinat, Cocoamphocarboxyglycinat, Dinatriumoleamido-MEA-sulfosuccinat, Aminglycinate, Aminpropionate sowie Aminsultaine, sowie deren Mischungen.

7. Shampoo nach Anspruch 5, wobei der Schaumbildner aus der Gruppe der Fettsäure mono- und - dialkanolamide bestehend aus Cocamid-MEA, Cocamid-DEA, Oleamid-MEA, Oleamid-DEA sowie deren Mischungen ausgewählt ist.

8. Shampoo nach Anspruch 5, wobei es sich bei dem Antioxidans um butyliertes Hydroxytoluol oder butyliertes Hydroxyanisol in einer Menge von ungefähr 0,01 bis ungefähr 1% (w/w) handelt.

9. Shampoo nach Anspruch 5, das weiterhin einen Haarbalsam enthält.

10. Shampoo nach Anspruch 5, das weiterhin ein oder mehrere Perlglanzmittel aus der Gruppe Ethylenglykoldistearat, Ethylenglykolmonostearat sowie deren Mischungen enthält.

11. Shampoo nach Anspruch 5, das weiterhin einen oder mehrere Duftstoffe und einen oder mehrere Farbstoffe enthält.

12. Verfahren zur Herstellung einer Shampooformulierung nach einem der vorhergehenden Ansprüche, umfassend die folgenden Schritte:

   (a) Erhitzen einer Lösung aus Verdickungsmittel und entionisiertem Wasser,
   (b) Vermischen der Tenside, des Schaumbildners und gegebenenfalls des Perlglanzmittels mit der Lösung von (a),
   (c) Vermischen des BHT mit der Lösung von (b),
   (d) Vermischen des Antimykotikums mit der Lösung von (c) ,
   (e) Dispergieren des Pyrithionsalzes in der Mischung von (d),
   (f) leichtes Abkühlenlassen der Suspension von (e) und Vermischen mit dem Konservierungsmittel bzw. den Konservierungsmitteln, dem Natriumchlorid zum Verdicken auf die erforderliche Viskosität, sowie gegebenenfalls dem Haarbalsam, dem Duft- und Farbstoff bzw. den Duft- und Farbstoffen,
   (g) Versetzen der Lösung von (f) mit Säure, Base oder Puffer zur Erzielung eines pH-Werts im Bereich von 4 bis 10, sowie
   (h) Versetzen der Lösung von (g) mit entionisiertem Wasser auf 100%.

**Revendications**

1. Composition lavante pour le corps ou les cheveux comprenant
   (a-1) du cétoconazole ou de l'élubiol en tant que premier ingrédient actif,
   (a-2) un sel de pyrithione en tant que deuxième ingrédient actif, et
   (b) des ingrédients de produits lavants pour le corps ou les cheveux connus dans la technique en tant que support,
   où les premier et deuxième ingrédients actifs sont présents en quantités produisant un effet synergique mutuel sur l'inhibition de la croissance de champignons dermatophytes.

2. Composition selon la revendication 1, dans laquelle le sel de pyrithione est la pyrithione de zinc ou la pyrithione sodique.

3. Composition selon la revendication 1 ou 2, dans laquelle le premier ingrédient actif est présent en une quantité s'échelonnant d'environ 0,1% à environ 2% (p/p) et le deuxième ingrédient actif est présent en une quantité s'échelonnant d'environ 0,05% à environ 2% (p/p), la quantité de ce dernier étant exprimée en poids de pyrithione de zinc.

4. Composition selon l'une quelconque des revendications précédentes, formulée en tant que shampooing.

5. Shampooing selon la revendication 4, dans lequel les ingrédients de shampooing connus dans la technique comprennent un ou plusieurs parmi un tensioactif, un agent moussant, un épaississant suffisant pour conférer à la formulation finale une viscosité dans la plage de 4000 à 9000 mPa.s à la température ambiante, un conservateur, un antioxydant, et un acide ou une base ou un tampon suffisant pour conférer au shampooing un pH dans la plage d'environ 4 à 10.

6. Shampooing selon la revendication 5, comprenant un ou plusieurs tensioactifs choisis parmi le groupe comprenant des sulfonates d'oléfines en C14-16 sodiques, le laurylsulfate sodique, le laureth-sulfate sodique, le cocoamidopropylaminoxyde, le laurylaminoxyde, le lauramido-DEA, la cocoamidopropylbétaïne, la lauryldiméthylbétaïne, la cocodiméthylsulfopropylbétaïne, le cocoylsarcosinate sodique, l'oléamido-MIPA-sulfosuccinate disodique, le cocoamido-MIPA-sulfosuccinate disodique, le laurethsulfosuccinate disodique, le cocoamphocarboxyglycinate, l'oléamido-MEA-sulfosuccinate disodique, des amineglycinates, des aminepropionates et des aminesultaïnes, et leurs mélanges.

7. Shampooing selon la revendication 5, dans lequel l'agent moussant est choisi parmi le groupe de mono- et dialcanolamides d'acides gras constitué du cocoamide-MEA, du cocoamide-DEA, de l'oléamide-MEA, de l'oléamide-DEA et de leurs mélanges.

8. Shampooing selon la revendication 5, dans lequel l'antioxydant est l'hydroxytoluène butylé ou l'hydroxyanisole butylé employé en une quantité d'environ 0,01 à environ 1% (p/p).

9. Shampooing selon la revendication 5, comprenant en outre un agent conditionneur.

10. Shampooing selon la revendication 5, comprenant en outre un ou plusieurs agents nacrants choisis parmi le groupe constitué du distéarate d'éthylèneglycol, du monostéarate d'éthylèneglycol et de leurs mélanges.

11. Shampooing selon la revendication 5, comprenant en outre un ou plusieurs parfums et un ou plusieurs colorants.

12. Procédé de préparation d'une formulation de shampooing telle que définie dans l'une quelconque des revendications précédentes, comprenant les étapes consistant à:

    (a) chauffer une solution d'épaississant et d'eau désionisée,

    (b) mélanger les tensioactifs, l'agent moussant et éventuellement l'agent nacrant avec la solution de (a),

    (c) mélanger le BHT avec la solution de (b),

    (d) mélanger l'agent antifongique avec la solution de (c),

    (e) disperser le sel de pyrithione dans le mélange de (d),

(f) laisser la suspension de (e) quelque peu refroidir et y mélanger le(s) conservateur(s), le chlorure de sodium destiné à un épaississement jusqu'à la viscosité requise, et éventuellement l'agent conditionneur, le(s) parfum (s) et le(s) colorant(s),

(g) ajouter un acide, une base ou un tampon à la solution de (f) pour donner lieu à un pH dans la plage de 4 à 10, et

(h) ajouter de l'eau désionisée à la solution de (g) jusqu'à 100%.